# EUROPEAN PATENT SPECIFICATION

(11) **EP 3 872 417 B1**
(45) Date of publication and mention of the grant of the patent: **31.05.2023**
(21) Application number: 19905260.6
(22) Date of filing: 07.03.2019
(51) Int. Cl.: F25B 7/00, F25B 43/00, A61B 18/02, F25B 9/00, F25B 9/10

(54) **PRECOOLING DEVICE FOR CRYOTHERAPY AND CRYOTHERAPY SYSTEM**
VORKÜHLVORRICHTUNG FÜR KRYOTHERAPIE UND KRYOTHERAPIESYSTEM
DISPOSITIF DE PRÉ-REFROIDISSEMENT POUR CRYOTHÉRAPIE ET SYSTÈME DE CRYOTHÉRAPIE

(30) Priority: 26.12.2018 CN 201811606020
(43) Date of publication of application: 01.09.2021
(73) Proprietor: Accu Target Medipharma (Shanghai) Co., Ltd., Shanghai 200000 (CN)
(72) Inventor: WU, Yinlong, Shanghai 201318 (CN); XU, Binkai, Shanghai 201318 (CN); YANG, Chi, Shanghai 201318 (CN); ZHANG, Rui, Shanghai 201318 (CN); CHANG, Kaiqiang, Shanghai 201318 (CN)
(74) Representative: EP&C
(86) International application number: PCT/CN2019/077260
(87) International publication number: WO 2020/133700

(56) References cited:
- CN-A- 101 584 602
- CN-A- 106 420 039

## Description

### TECHNICAL FIELD

The present invention relates to the field of medical treatment, in particular to a pre-cooling device for cryotherapy and a cryotherapy system.

### BACKGROUND

As a minimally invasive targeted surgery, cryoablation has the characteristics of less trauma, less toxic and side effects and a definite curative effect. It also has the advantages of achieving clear boundaries of ablation ice balls, participating in activating the body's tumor immune function, causing no damage to large blood vessels and causing no obvious pain. Therefore, it realizes practical application of ultralow-temperature targeted cryotherapy and hyperthermia for tumors. In recent years, cryosurgery has been widely used in treatment of a metastatic liver cancer, a prostate cancer, and a kidney cancer. Wherein, helium, or nitrogen at a normal temperature and under a high pressure such as a pressure of 5 MPa to 10 MPa is used as a working gas for an operation.

In patents with publication numbers of CN107951558A and CN106420039 A, in order to achieve multi-functional pre-cooling of a gas, a variety of pre-cooling demands is met through multi-level control. However, the pressure of a pre-cooled gas is usually set manually and various types of decompression valves are required for regulating different pressures, which is not conducive to realizing targeted timely and stable regulation.

### SUMMARY

The present invention provides a pre-cooling device for cryotherapy and a cryotherapy system according to the independent claim to solve the problem about a difficulty in realizing targeted timely and stable regulation because the pressure of a pre-cooled gas is usually set manually and various types of decompression valves are required for regulating different pressures.

According to a first aspect of the present invention, a pre-cooling device for cryotherapy is provided and comprises a gas inlet port, at least three vent branches, a single-stage-compression auto-cascade refrigeration system assembly and a pressure regulating assembly, wherein the gas inlet port communicates with a gas inlet end of the pressure regulating assembly; a gas outlet end of the pressure regulating assembly communicates with gas inlet ends of the at least three vent branches respectively; a gas inlet of a cryoprobe connector for a cryoprobe communicates with gas outlet ends of the at least three vent branches respectively;
the single-stage-compression auto-cascade refrigeration system assembly comprises at least three-stage refrigeration cycles; for at least some of the refrigeration cycles, a refrigeration temperature thereof is lower than that of the previous stage refrigeration cycle;
each refrigeration cycle is capable of exchanging heat with a gas flowing in the corresponding vent branch to pre-cool the gas flowing in this vent branch; each vent branch is provided with a branch control member configured to control connection and disconnection of this vent branch; and
the pressure regulating assembly is configured to control the pressure of a gas outlet end of the pressure regulating assembly, the pressure of the gas outlet end of the pressure regulating assembly is associated with a vent branch to be pre-cooled, and for at least two vent branches, the gas outlet end of the pressure regulating assembly has different pressures during pre-cooling.

Optionally, when a temperature represented by the second temperature information is equal to a required temperature and the pressure of the gas outlet end of the pressure regulating assembly reaches a target pressure, the pressure regulating assembly may be configured to control the pressure of the gas outlet end of the pressure regulating assembly to be changed according to a change of the first temperature information, and maintain a temperature represented by the second temperature information to be equal to the required temperature.

The pressure regulating assembly is configured to control the pressure of the gas outlet end of the pressure regulating assembly according to first pressure information and/or second pressure information, wherein the first pressure information represents a pressure of the gas inlet end of the cryoprobe connector, and the second pressure information represents a pressure of the gas inlet end of the pressure regulating assembly.

The pressure regulating assembly includes a proportional decompression valve and a controller; the gas inlet end of the cryoprobe connector is connected with a first pressure sensor configured to detect the first pressure information; the inlet end of the proportional decompression valve is connected with a second pressure sensor configured to detect the second pressure information; the gas outlet end of the proportional decompression valve is connected with a third pressure sensor; and
the controller is connected with the first pressure sensor, the second pressure sensor and the third pressure sensor respectively, and is configured to control the proportional decompression valve according to information detected by the first pressure sensor, the second pressure sensor and the third pressure sensor.

Optionally, the pressure regulating assembly may be configured to control the pressure of the gas outlet end of the pressure regulating assembly according to the temperature information; the temperature information includes first temperature and/or second temperature information; the first temperature information represents temperatures of the vent branches; and the second temperature information represents a temperature of the gas inlet end of the cryoprobe connector.

Optionally, the first temperature information may represent a temperature of a gas refrigerated by the corresponding refrigeration cycle in the corresponding vent branch; and
after a temperature represented by the second temperature information is equal to a required temperature and the pressure of the gas outlet end of the pressure regulating assembly reaches a target pressure, the pressure regulating assembly may be configured to control the pressure of the gas outlet end of the pressure regulating assembly to be changed according to a change of the first temperature information, and maintaining the temperature represented by the second temperature information to be equal to the required temperature.

Optionally, in a control mode, if a temperature represented by the temperature information does not reach a preset target temperature, the pressure regulating assembly may be configured to control the pressure of the gas outlet end of the pressure regulating assembly to be matched with the first target pressure information corresponding to a vent branch to be pre-cooled; and after the temperature represented by the temperature information reaches the target temperature and is kept for a preset target time, the pressure of the gas outlet end of the pressure regulating assembly is controlled to drop to a value matched with the second target pressure information of the vent branch to be pre-cooled.

Optionally, the pre-cooling device further includes a return gas pre-cooling assembly; the return gas pre-cooling assembly comprises a gas return channel and a return gas pre-cooling control member which is arranged in the gas return channel; a gas inlet end of the gas return channel communicates with a return gas end of the cryoprobe connector; and a gas circulating in the gas return channel can exchange heat with a gas circulating in the at least three vent branches to pre-cool the gas circulating in the at least three vent branches.

Optionally, the gas circulating in the vent branches first exchanges heat with the gas circulating in the gas return channel to achieve first pre-cooling, and then exchange heat with the corresponding refrigeration cycle to achieve second pre-cooling.

Optionally, in at least some of the refrigeration cycles, a working medium for pre-cooling in each refrigeration cycle may be obtained by cooling a working medium for pre-cooling in the previous stage refrigeration cycle.

Optionally, each of the at least some of the refrigeration cycles may be provided with a condensation evaporator and a gas-liquid separator;
excluding the first one of the at least three-stage refrigeration cycles, the gas-liquid separators of the other refrigeration cycles are all used for gas and liquid separation of a gaseous working medium discharged from the previous stage refrigeration cycle to realize cooling; and the condensation evaporator of each stage of the other refrigeration cycles is configured to control a gaseous working medium discharged from the gas-liquid separator of the same stage refrigeration cycle, a gas circulating in the corresponding vent branch and a liquid working medium discharged from the gas-liquid separator in this corresponding refrigeration cycle to exchange heat with one another.

Optionally, the first refrigeration circle may be provided with a compressor, a condenser, a condensation evaporator and a gas-liquid separator, and the last stage of the at least three-stage refrigeration cycles is provided with an evaporator.

A first input end and a first output end of the evaporator are connected to the corresponding vent branch; a second input end of the evaporator is connected with a gas outlet of the gas-liquid separator of the previous stage refrigeration cycle; and a second output end of the evaporator is connected with a first inlet of the condensation evaporator of the previous stage refrigeration cycle.

Excluding the first stage refrigeration cycle and the last stage refrigeration cycle, the inlet of the gas-liquid separator of each stage of the other refrigeration cycles communicates with a second outlet of the condensation evaporator of the previous stage refrigeration cycle; a gas outlet of the gas-liquid separator of each stage of the other refrigeration cycles communicates with a second inlet of the condensation evaporator of the same stage refrigeration cycle; a liquid outlet of the gas-liquid separator of each stage of the other refrigeration cycles communicates with a first inlet of the condensation evaporator of the same stage refrigeration cycle; a first outlet of the condensation evaporator of each stage of the other refrigeration cycles communicates with a first inlet of the condensation evaporator of the previous stage refrigeration cycle; a third inlet and a third outlet of the condensation evaporator of each stage of the other refrigeration cycles are respectively connected into the corresponding vent branch;
the outlet of the compressor communicates with the inlet of the condenser; the outlet of the condenser communicates with the inlet of the gas-liquid separator of the first stage refrigeration cycle; the liquid outlet of the gas-liquid separator of the first stage refrigeration cycle communicates with the first inlet of the condensation evaporator of the same stage refrigeration cycle; a gas outlet of the gas-liquid separator of the first stage refrigeration cycle communicates with the second inlet of the condensation evaporator of the same stage refrigeration cycle; the first outlet of the condensation evaporator of the first stage refrigeration cycle communicates with the inlet of the compressor; and the third inlet and the third outlet of the condensation evaporator of the first stage refrigeration cycle are respectively connected into the corresponding vent branch.

Optionally, a bypass pipeline may be arranged between the gas outlet of the gas-liquid separator of the first stage refrigeration cycle and the inlet of the compressor; the bypass pipeline is provided with an expansible vessel and a bypass control member; and the bypass control member is arranged at one side of the inlet and/or one side of the outlet of the expansible vessel.

According to a second aspect of the present invention, a cryotherapy system is provided and comprises a cryoprobe and a pre-cooling device for cryotherapy according to the first aspect as well as its relevant alternatives, wherein the pre-cooling device communicates with the cryoprobe.

The pre-cooling device for cryotherapy and the cryotherapy system provided by the present invention may realize precooling to multiple temperatures of different levels by virtue of a single-stage-compression auto-cascade refrigeration system assembly, wherein the single-stage-compression auto-cascade refrigeration system assembly includes at least three-stage refrigeration cycles. Therefore, the pre-cooling device and the cryotherapy system can meet a variety of requirements of different operations and different gases.

Also, for the precooling to temperatures of different levels, the present invention may realize control on the pressure of the gas outlet end of the pressure regulating assembly by virtue of the pressure-regulating assembly. Moreover, the pressure of the gas outlet end of the pressure regulating assembly is associated with the vent branch to be pre-cooled. Therefore, during pre-cooling of different levels, a ventilation pressure of the pre-cooling device is automatically and timely controlled, and the stability of the pressure is maintained as well. In addition, by automatic regulation of the ventilation pressure, it is beneficial to increase the refrigeration speed through a matched ventilation pressure, thereby helping to avoid the problem about a too long pre-cooling time.

In an optional solution of the present invention, the ventilation pressure of the pre-cooling device may be regulated according to different current temperatures and regulation demands in conjunction with changes of the temperature information.

Further, when the temperature represented by the temperature information does not reach a target temperature, the pressure of the gas outlet end of the pressure regulating assembly may be controlled to be matched with the first target pressure information; when the temperature represented by the temperature information reaches the target temperature, the pressure is controlled to drop to a value matched with the second target pressure information; and therefore a gas is effectively saved under a condition that the gas reaches a required temperature.

In an optional solution of the present invention, double times of pre-cooling is also realized by a return gas pre-cooling assembly and a single-stage-compression auto-cascade refrigeration system assembly, so that the refrigeration temperature may be further reduced and the refrigeration speed may be effectively increased.

In an optional solution of the present invention, multi-stage refrigeration cycles are formed by gas-liquid separators and gas condensation evaporators, so that multi-stage refrigeration in a wide temperature range is realized, the refrigeration speed may be effectively increased as well, and the problem about a too long pre-cooling time is avoided.

### BRIEF DESCRIPTION OF THE DRAWINGS

To describe examples of the present invention or technical solutions in the prior art more clearly, the drawings required in description of the examples or the prior art will be briefly described below. Apparently, the drawings described below are merely some examples of the present invention, and other drawings can also be obtained by those skilled in the art based on these drawings without creative efforts.
Fig. 1 is a structure diagram of a pre-cooling device for cryotherapy in one example of the present invention;
Fig.2 is a structure diagram of a pre-cooling device for cryotherapy in another example of the present invention;
Fig.3 is a structure diagram of a pressure regulating assembly in one example;
Fig.4 is a structure diagram of a pre-cooling device for cryotherapy in another example of the present invention; and
Fig.5 is a structure diagram of a single-stage-compression auto-cascade refrigeration system assembly in one example of the present invention.

Description of reference symbols:
1-Pressure Regulating Assembly;
10 1 -Controller;
102-Proportional Decompression Valve;
103-First Pressure Sensor;
104-Second Pressure Sensor;
105-Third Pressure Sensor;
106-First Temperature Sensor;
107-Second Temperature Sensor;
108-Third Temperature Sensor;
2-Single-Stage-Compression Auto-Cascade Refrigeration System Assembly;
200-Refrigeration Cycle;
201-Gas-liquid separator;
202-Condensation Evaporator;
203 -Evaporator;
204-Compressor;
205 -Condenser;
206-Expansible Vessel;
207-Bypass Control Member;
208-Capillary Tube;
209- Filter Drier;
3-Branch Control Member;
4-CryoprobeConnector;
5-Reheater;
6-Return Gas Pre-cooling Control Member;
7-First Maintenance Control Member;
8-Gas Inlet Port;
9-Main Control Member;
10-Filter Drier;
11-Second Maintenance Control Member.

### DETAILED DESCRIPTION

Technical solutions in the examples of the present invention will be clearly and completely described below in conjunction with the drawings in the examples of the present invention. Apparently, the examples described are merely some examples rather than all examples of the present invention. Based on the examples of the present invention, all other examples obtained by those skilled in the art without any creative efforts shall fall within the protection scope of the present invention.

In the description, the claims and the drawings of the present invention, the terms "first", "second", "third", "fourth", etc. (if existing) are used for distinguishing similar objects rather than describing a specific order or sequence. It should be understood that the data used in this way may be interchanged under appropriate circumstances so that the examples of the present invention described herein may be implemented in an order other than those illustrated or described herein. In addition, the terms "including" and "comprising" and any variations thereof are intended to cover non-exclusive inclusions. For example, a process, method, system, product, or device that includes a series of steps or units is not necessarily limited to those clearly listed steps or unit, but may also include other steps or unit that are not clearly listed or are inherent to the process, method, product, or device.

The technical solutions of the present invention will be described in detail below with specific examples. The following specific examples may be combined with each other, and the same or similar concepts or processes may not be repeated in some examples.

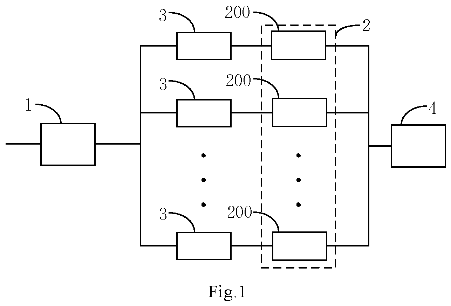

Fig. 1 is a structure diagram of a pre-cooling device for cryotherapy in one example of the present invention.

As shown in Fig. 1, the pre-cooling device for cryotherapy includes a gas inlet port, at least three vent branches, a single-stage-compression auto-cascade refrigeration system assembly 2 and a pressure regulating assembly 1. The gas inlet port communicates with the gas inlet end of the pressure regulating assembly 1; the gas outlet end of the pressure regulating assembly 1 communicates with the gas inlet ends of the at least three vent branches respectively; and the gas inlet end of a cryoprobe connector 4 for a cryoprobe communicates with the gas outlet ends of the at least three vent branches.

The single-stage-compression auto-cascade refrigeration system assembly 2 may be considered as any assembly that uses a single-stage-compression auto-cascade refrigeration system to pre-cool a gas circulating in the vent branches. The single-stage-compression auto-cascade refrigeration system assembly 2 may include at least three-stage refrigeration cycles 200. The refrigeration cycles 200 may be considered as achieving a refrigeration effect by circulation of a working medium. Each refrigeration cycle may be an auto-cycle. All refrigeration cycles also do not need to form an independent auto-cycle respectively but may be combined to form a continuous overall cycle; and each refrigeration cycle may refer to a procedure where refrigeration occurs in the overall cycle.

For at least some of the refrigeration cycles, a refrigeration temperature thereof is lower than that of the previous stage refrigeration cycle; therefore, the precooling to temperatures of different levels may be realized by multi-stage refrigeration cycles; and as a result, the pre-cooling device may meet a variety of demands of different operations and gases. For example, the following four levels of regulation may be realized: -20°C, -60°C, -80°C, -120°C. The four levels may be considered as different boiling points of a working gas. The levels are distinguished for matching the pre-cooling device with working gases with different boiling points so as to realize a plurality of functions by one machine. It can be seen that different branches may correspond to different temperatures, and may also correspond to different working gases.

Therefore, refrigeration cycles of different levels are provided in this example; meanwhile, the demands of different gases and temperatures are met by regulation of single-channel gas pressure; and thus, refrigeration integration of different gases and temperatures is realized.

In this example, a plurality of vent branches may be connected into the gas inlet port and the pressure regulating assembly 1, and may also be connected to the cryoprobe connector 4. It can be seen that the present invention may achieve single-channel gas input and output. Compared with a way of adopting multi-channel input or output in the prior art, this example is more compact and smaller and meets the requirements of an operation environment of a hospital.

In this example, each refrigeration cycle may exchange heat with a gas circulating in the corresponding vent branch to pre-cool the gas circulating in this vent branch, and each vent branch is provided with a branch control member 3 configured to control connection and disconnection of this vent branch. The branch control member 3 may be a single assembly or a combination of a plurality of members, where the combination may be, for example, serial connection and/or parallel connection. In one embodiment, the branch control member 3 may include a solenoid valve and a one-way valve, and the solenoid valve may be a normally closed solenoid valve.

In this example, the pressure regulating assembly 1 may be configured to control the pressure of the gas outlet end of the pressure regulating assembly; the pressure of the gas outlet end of the pressure regulating assembly is associated with a vent branch to be pre-cooled; and for at least two vent branches, the gas outlet end of the pressure regulating assembly has different pressures during pre-cooling.

Specifically, the pressure regulating assembly is specifically configured to, after the vent branch to be pre-cooled is determined, determine target pressure information of the gas outlet end of the pressure regulating assembly according to a preset corresponding relation between the vent branch and pressure information, and control the pressure of the gas outlet end of the pressure regulating assembly 1 according to the target pressure information.

The above-mentioned corresponding relation may be pre-configured based on the requirements of operations adopting different gases as well as the requirements generated based on this at different temperatures. The above-mentioned corresponding relation may be used in this example.

In this example, the pressure of the gas outlet end of the pressure regulating assembly 1 may be considered as a working pressure that is associated with the vent branch to be pre-cooled. In other words, the working pressure is controlled based on the current vent branch where it works for. Wherein, different vent branches correspond to different refrigeration temperatures, so it may also be understood as that the pressure of the gas outlet end of the pressure regulating assembly 1, namely the working pressure is determined based on the level of the refrigeration temperature for the current required refrigeration.

It can be seen that according to the precooling to temperatures of different levels, this example may control the pressure of the gas outlet end of the pressure regulating assembly by virtue of the pressure regulating assembly. Moreover, the pressure of the gas outlet end of the pressure regulating assembly is associated with a vent branch to be pre-cooled. Therefore, during pre-cooling of different levels, a ventilation pressure of the pre-cooling device is controlled automatically and timely, and the stability of the pressure is maintained. In addition, by automatic regulation of the ventilation pressure, it is beneficial to increase the refrigeration speed through a matched ventilation pressure, thereby helping to avoid the problem about a too long pre-cooling time.

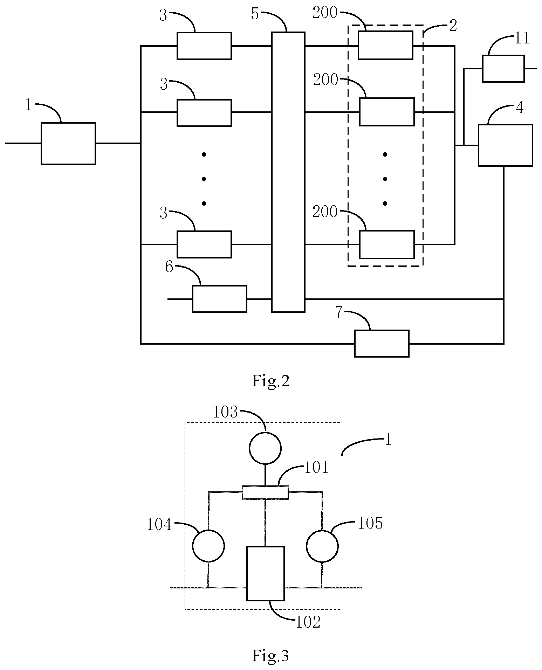

Fig. 2 is a structure diagram of a pre-cooling device for cryotherapy in another example of the present invention.

As shown in Fig. 2, the pre-cooling device further includes a return gas pre-cooling assembly. The return gas pre-cooling assembly includes a gas return channel and a return gas pre-cooling control member 6 that is arranged in the gas return channel. A gas inlet end of the gas return channel communicates with a return gas end of the cryoprobe connector 4. Gas circulating in the gas return channel may exchange heat with the gas circulating in the at least three vent branches to pre-cool the gas circulating in the at least three vent branches. In a specific implementation process, the heat exchange may be realized through a reheater 5.

Wherein, the return gas pre-cooling control member 6 may be considered as any member or combination of members that may control whether the gas return channel circulates gas with the outside or not. The return gas pre-cooling control member 6 may be a solenoid valve, specifically a normally open solenoid valve.

In a specific implementation process, the gas circulating in the vent branches may first exchange heat with the gas circulating in the gas return channel to achieve first pre-cooling, and then exchange heat with the corresponding refrigeration cycle to achieve second pre-cooling. In addition, the gas return channel may communicate with external air, and thus, outward discharging of recycled gas may be facilitated when the return gas pre-cooling control member 6 controls the circulation of the gas return channel.

Meanwhile, during discharging, the recycled gas may be further utilized to exchange heat with the vent branches, thereby helping to further reduce the refrigeration temperature and effectively increase the refrigeration speed.

In one embodiment, the pre-cooling device may further include a first maintenance channel and a second maintenance channel. One end of the first maintenance channel is connected with the gas outlet end of the pressure regulating assembly 1, and the other end is connected with the gas return end of the cryoprobe connector 4. The first maintenance channel is provided with a first maintenance control member 7. The second maintenance channel is connected with the gas inlet end of the cryoprobe connector 4, and is provided with a second maintenance control member 11.

Thus, by the control of the first maintenance control member 7 and the second maintenance control member 11, a gas enters the first maintenance channel through the pressure regulating assembly 1, subsequently enters the gas return end of the cryoprobe connector 4, and finally enters the cryoprobe; the cryoprobe returns the gas through the gas inlet end; and specifically the gas is discharged into the second maintenance channel through the gas inlet end, and discharged into air through the second maintenance channel. The first maintenance channel and the second maintenance channel are applied to gas protection in electric reheating, to prevent an electric heating wire from damaging internal assemblies of the cryoprobe.

In a specific implementation process, the first maintenance control member 7 may include a solenoid valve and a one-way valve, and the solenoid valve may be a normally closed valve. The second maintenance control member11 may include a solenoid valve, which may also be a normally closed valve.

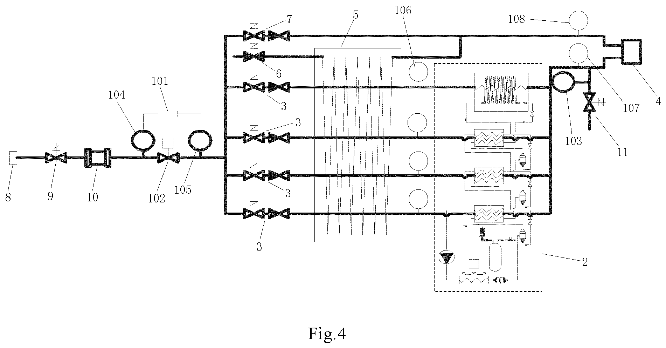

Fig. 3 is a structure diagram of a pressure regulating assembly in one example of the present invention. Fig. 4 is a structure diagram of a pre-cooling device for cryotherapy in another example of the present invention.

As shown in Fig. 3 and Fig. 4, the pressure regulating member 1 is specifically configured to control the pressure of the gas outlet end of the pressure regulating assembly 1 according to the first pressure information and/or the second pressure information.

The first pressure information may represent the pressure of the gas inlet end of the cryoprobe connector 4, and specifically the pressure may be detected by a first pressure sensor 103 connected to the gas inlet end of the cryoprobe connector 4.

The second pressure information may represent the pressure of the gas inlet end of the pressure regulating assembly 1, and specifically, the pressure may be detected by a second pressure sensor 104 at a corresponding position.

In addition, the first pressure information and the second pressure information may be real-time numerical values, or may be calculated statistic data.

Controlling the pressure of the gas outlet end of the pressure regulating assembly 1 may mean controlling the pressure to be a certain specific pressure, or controlling the pressure within a certain specific pressure range.

The first pressure information and/or the second pressure information may provide a basis for control of a required working pressure. Based on the information, any control method such as PID control may be used, a controller such as a PIC controller may be configured for control. Thus, the working pressure is controlled timely, effectively and accurately.

In one embodiment, as shown in Fig. 3 and Fig. 4, the pressure regulating assembly 1 may include a proportional decompression valve 102 and a controller 101. The gas inlet end of the cryoprobe connector 4 is connected with a first pressure sensor 103 configured to detect the first pressure information. The gas inlet end of the proportional decompression valve 102 is connected with a second pressure sensor 104 configured to detect the second pressure information. The gas outlet end of the proportional decompression valve 102 is connected with a third pressure sensor 105.

The controller 101 is connected with the first pressure sensor 103, the second pressure sensor 104 and the third pressure sensor 105 respectively. This connection may be feasible communication, namely including various situations such as wired direct connection, wired indirect connection, wireless direct connection and wireless indirect connection. The controller 101 is configured to control the proportional decompression valve 102 according to information detected by the first pressure sensor 103, the second pressure sensor 104 and the third pressure sensor 105. A specific control method of the controller 101 and factors to be considered for control is changed according to the description of any implementation way of this example, and the description for representing the factors considered may include the information detected by the above three pressure sensors.

In a specific implementation process, the controller may be configured to determine a vent branch to be pre-cooled, determine target pressure information of the gas outlet end of the pressure regulating assembly according to a preset corresponding relation between this vent branch and the pressure information, and control the pressure of the gas outlet end of the pressure regulating assembly according to at least one of the above related first pressure information, second pressure information and third pressure information.

Wherein, the process of determining the vent branch to be pre-cooled may be, for example, the controller is connected with the branch control member of each vent branch, thus a signal may be sent to the controller when the corresponding branch control member is connected and/or disconnected, and finally the controller may determine the vent branch to be pre-cooled according to the received signal.

Based on different control algorithms, the information detected by the pressure sensors may also be calculated in various ways. In addition, some of the information may not be used for direct control, and may further be configured to regulate and verifying control results. It can be seen that any method for applying the detected information shall not depart from the scope described in this example.

In one embodiment, in a control mode, circulation in a corresponding vent branch may be controlled after a current required pre-cooling temperature is determined, thus the pressure of the gas outlet end of the pressure regulating assembly is controlled to be matched with the pre-cooling temperature or the corresponding vent branch, which may be considered that the pressure of the gas outlet end of the pressure regulating assembly is controlled to be matched with target pressure information corresponding to the vent branch to be pre-cooled.

The target pressure information may refer to a specific pressure or a specific pressure range.

In a specific implementation process, the target pressure information may be, for example, a pressure of 1500 PSI or 1200 PSI, or a pressure range of 500 PSI to 700 PSI. Wherein, the PSI may be considered as pound force per square inch.

Wherein, 1 PSI = 6894,76 Pa.

In a specific example, when a conventional nitrogen or argon source is used as a working gas, different ultralow-temperature pre-cooling nay be realized in an ultralow-temperature pre-cooling control mode in combination with the above related two times of pre-cooling.

In one example, a gas enters the pressure regulating assembly 1, passes through the reheater 5 after the working pressure is regulated to about 1500 PSI, enters the single-stage-compression auto-cascade refrigeration system assembly again after pre-cooled once to be subjected to second pre-cooling in one of the refrigeration cycles, is accordingly pre-cooled to -120°C or less, and finally enters the cryoprobe via the cryoprobe connector 4. Thus, a cutter bit may reach a temperature of -150°C or less, and the returned gas flows through the reheater 5 via the gas return end and is discharged to air after heated. The gas may refer to conventional industrial nitrogen or argon.

In another example, a gas enters the pressure regulating assembly 1, passes through the reheater 5 after the working pressure is regulated to about 1500 PSI, enters the single-stage-compression auto-cascade refrigeration system assembly again after pre-cooled once to be subjected to second pre-cooling in another one of the refrigeration cycles, is accordingly pre-cooled to -60°C or less, and finally enters the cryoprobe via the cryoprobe connector 4. Thus, a cutter bit can reach a temperature of -80°C or less, and the returned gas flows through the reheater 5 via the gas return end and is discharged to air after heated. The gas may also refer to high-pressure argon or conventional nitrogen monoxide.

In another example, a gas enters the pressure regulating assembly 1, passes through the reheater 5 after the working pressure is regulated to about 500 PSI to 700 PSI, enters the single-stage-compression auto-cascade refrigeration system assembly again after pre-cooled once to be subjected to second pre-cooling in another one of the refrigeration cycles, is accordingly pre-cooled to -20°C or less, and finally enters the cryoprobe via the cryoprobe connector 4. Thus, a cutter bit reaches a temperature of -40°C or less, and the returned gas flows through the reheater 5 via the gas return end and is discharged to air after heated. The gas may refer to conventional carbon dioxide or nitrous oxide.

In one embodiment, the pressure regulating assembly 1 may be specifically configured to control the pressure of the gas outlet end of the pressure regulating assembly 1 according to temperature information and the vent branch to be pre-cooled.

The temperature information may include the first temperature information and/or the second temperature information. The first temperature information may represent a temperature of the corresponding vent branch and may be measured by a first temperature sensor 106 arranged in the corresponding vent branch. The second temperature information may represent a temperature of the cryoprobe connector 4 and may be measured by a second temperature sensor 107 arranged at the gas inlet end of the cryoprobe connector 4. In addition, a third temperature sensor 108 may also be configured to measure the temperature of the gas return end of the cryoprobe connector 4.

Based on control of temperature information, more various basis may be provided for implementation of control to ensure that the control fits for the current temperature situation.

The first temperature information may specifically represent a temperature of the gas refrigerated by the corresponding refrigeration cycle in the vent branch. Therefore, the first temperature sensor 106 configured to detect the first temperature information may be arranged at one side of the front end of the corresponding refrigeration cycle in the single-stage-compression auto-cascade refrigeration system assembly 2 shown in Fig. 4, or may be arranged at one side of the rear end, to detect the temperature after refrigeration, namely the first temperature information.

After the temperature represented by the second temperature information is equal to a required temperature and the pressure of the gas outlet end of the pressure regulating assembly reaches a target pressure, the pressure regulating assembly may be further configured to control the pressure of the gas outlet end of the pressure regulating assembly to be changed according to a change of the first temperature information, and maintaining the temperature represented by the second temperature information to be equal to the required temperature.

The change of the pressure may be, for example: the pressure may be reduced when the temperature represented by the first temperature information is reduced and may be increased when the temperature represented by the first temperature information is increased.

In one embodiment, the pressure regulating assembly 1 is specifically configured to, in one control mode, control the pressure of the gas outlet end of the pressure regulating assembly 1 to be matched with first target pressure information corresponding to the vent branch to be pre-cooled when a temperature represented by the temperature information does not reach a preset target temperature, and control the pressure of the gas outlet end of the pressure regulating assembly 1 to drop to a value matched with second target pressure information corresponding to the vent branch to be pre-cooled when the temperature represented by the temperature information reaches the target temperature and is kept for a preset target time.

Wherein, the first target pressure information and the second target pressure information may refer to a specific pressure or a specific pressure range, and also the pressure represented by the second target pressure information shall be lower than that represented by the first target pressure information.

If the first target pressure information and the second target pressure information both represent a pressure, the pressure represented by the second target pressure information shall be lower than that represented by the first target pressure information.

If the first target pressure information represents a pressure and the second target pressure information represents a pressure range, the upper limit of the pressure range represented by the second target pressure information is lower than the pressure represented by the first target pressure information.

If the first target pressure information represents a pressure range and the second target pressure information represents a pressure, the lower limit of the pressure range represented by the first target pressure information is higher than the pressure represented by the second target pressure information.

If the first target pressure information and the second target pressure information both represent a pressure range, the lower limit of the pressure range represented by the first target pressure information is higher than the upper limit of the pressure range represented by the second target pressure information in one specific implementation process. In other specific implementation processes, it may also be understood as that a statistical value such as an average value of the pressure range represented by the first target pressure information is higher than that of the pressure range represented by the second target pressure information.

The above control mode may be a gas saving mode.

The target temperature may be, for example, a minimum temperature of -150°C. The target time may be, for example, 3 min. In a specific example, gas enters the pressure regulating assembly 1, passes through the reheater 5 after the working pressure is regulated to about 1500 PSI, enters the single-stage-compression auto-cascade refrigeration system assembly again after pre-cooled once to be subjected to second pre-cooling, and finally enters the cryoprobe through the cryoprobe connector 4; at the moment, a cryoprobe tip starts to be cooled, after it reaches a minimum temperature (such as -150°C) and is kept at the minimum temperature for 3 min, the controller of the pressure regulating assembly 1 may slowly reduce the working pressure of the system, for example, to 1200 PSI in an automatically-controlled gas saving mode; thus the flow rate of the system is reduced, but the cryoprobe tip can still be kept at the minimum temperature; by this operation, small influence is caused to the cooling capacity of the cryoprobe tip; and as a result, the amount of consumed gas may be reduced.

It can be seen that in this embodiment, the ventilation pressure of the pre-cooling device may be regulated according to different current temperatures and regulation demands in combination with the changes of the temperature information. Further, when the temperature represented by the temperature information does not reach a target temperature, the pressure of the gas outlet end of the pressure regulating assembly is controlled to be matched with the first target pressure information; when the temperature represented by the temperature information reaches the target temperature, the pressure is controlled to drop to a value matched with the second target pressure information; and therefore a gas is effectively saved under a condition that the gas reaches a required temperature.

In addition, the pressure regulating assembly 1 may also control the pressure according to a pressure detected by the first pressure sensor 103 and the third pressure sensor 104 to ensure that the pressure difference is within a safe range.

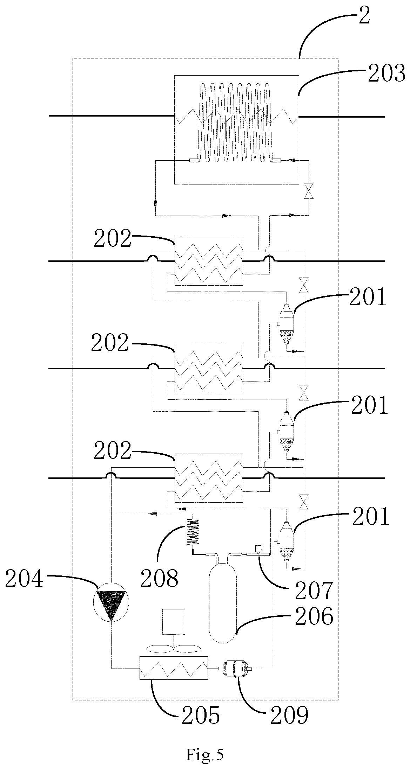

Fig. 5 is a structure diagram of a single-stage-compression auto-cascade refrigeration system assembly in one example of the present invention.

As shown in Fig. 4 and Fig. 5, a working medium for pre-cooling in each of the at least some of the refrigeration cycles is obtained by cooling a working medium for pre-cooling in the previous stage refrigeration cycle.

In one embodiment, each of the at least some of the refrigeration cycles is provided with a condensation evaporator 202 and a gas-liquid separator 201.

The at least some of the refrigeration cycles may refer to all refrigeration cycles excluding the last one of the at least three-stage refrigeration cycles, or may refer to all refrigeration cycles excluding the first one and the last one of the at least three-stage refrigeration cycles, or may refer to all refrigeration cycles.

Excluding the gas-liquid separator of the last stage of the at least three-stage refrigeration cycles, the gas-liquid separator 201 of each stage of the other refrigeration cycles is used for gas and liquid separation of a gaseous working medium discharged from the gas-liquid separator 201 of the previous stage refrigeration cycle to implement the pre-cooling; the condensation evaporator 202 of each stage of the other refrigeration cycles is configured to control heat exchange among a gaseous working medium discharged from the gas-liquid separator 201 of the same stage refrigeration cycle, a gas circulating in the corresponding vent branch and a liquid working medium discharged from the gas-liquid separator of the same stage refrigeration cycle; thus, the gaseous working medium discharged from the gas-liquid separator 201 may facilitate cooling of the gas circulating in the corresponding vent branch and cooling of the liquid working medium discharged from the gas-liquid separator 201 of the same stage refrigeration cycle; and as a result, the liquid working medium discharged from the gas-liquid separator 201 is evaporated into a gaseous working medium.

In this embodiment, multi-stage refrigeration cycles are formed by gas-liquid separators and gas condensation evaporators, so that multi-stage refrigeration in a wide temperature range is realized, the refrigeration speed is effectively increased as well, and the problem about a too long pre-cooling time is avoided. Specifically, gas and liquid separation is realized by gas-liquid separators, and subsequently mixed working mediums are driven to flow in condensation evaporators through throttling refrigeration, thereby achieving a wide temperature range from an evaporation temperature of-40°C of a conventional refrigeration cycle to an ultra-low temperature of -180°C. Also, different pre-cooling temperatures is achieved according to the temperature differences of heat exchangers in different stages.

In one embodiment, the first stage refrigeration cycle is provided with a compressor 204, a condenser 205, a condensation evaporator 202 and a gas-liquid separator 201, and the last stage of the at least three-stage refrigeration cycles is provided with an evaporator 203.

As shown in Fig. 5, a first input end and a first output end of the evaporator 203 are connected into a corresponding vent branch; a second input end of the evaporator 203 is connected with a gas outlet of the gas-liquid separator 201 of the previous stage refrigeration cycle; and a second output end of the evaporator 203 is connected with a first inlet of the condensation evaporator 202 of the previous stage refrigeration cycle.

Excluding the first stage refrigeration cycle and the last stage refrigeration cycle, the inlet of the gas-liquid separator 201 of each stage of the other refrigeration cycles communicates with the second outlet of the condensation evaporator 202 of the previous stage refrigeration cycle; the gas outlet of the gas-liquid separator 201 of each stage of the other refrigeration cycles communicates with the second inlet of the condensation evaporator 202 of the same stage refrigeration cycle; the liquid outlet of the gas-liquid separator 201 of each stage of the other refrigeration cycles communicates with the first inlet of the condensation evaporator 202 of the same stage refrigeration cycle; the first outlet of the condensation evaporator 202 in each of the refrigeration cycles communicates with the first inlet of the condensation evaporator 202 of the previous stage refrigeration cycle; and the third inlet and the third outlet of the condensation evaporator 202 of each stage of the other refrigeration cycles are respectively connected into the corresponding vent branch.

The outlet of the compressor 204 communicates with the inlet of the condenser 205. The outlet of the condenser 205 communicates with the inlet of the gas-liquid separator 201 of the first stage refrigeration cycle. The liquid outlet of the gas-liquid separator 201 of the first stage refrigeration cycle communicates with the first inlet of the condensation evaporator 202 of the same stage refrigeration cycle. The gas outlet of the gas-liquid separator 201 of the first stage refrigeration cycle communicates with the second inlet of the condensation evaporator 202 of the same stage refrigeration cycle. The first outlet of the condensation evaporator 202 of the first stage refrigeration cycle communicates with the inlet of the compressor 204. The third inlet and the third outlet of the condensation evaporator 202 of the first stage refrigeration cycle are respectively connected into the corresponding vent branch.

Taking Fig. 5 as an example, the first inlet and the first outlet of each condensation evaporator 202 may be a pair of inlets and outlets drawn at the bottom of the condensation evaporator 202 shown in the figure; the second inlet and the second outlet of each condensation evaporator 202 may be a pair of inlets and outlets drawn on the top of the condensation evaporator 202 shown in the figure; and the third inlet and the third outlet of each condensation evaporator 202 may be a pair of inlets and outlets in the middle of the condensation evaporator 202 shown in the figure.

Through the above connection method, a multi-stage single-stage-compression auto-cascade refrigeration system is achieved, which meets the requirements of multi-level refrigeration as well as effectively improves the refrigeration efficiency and shortens the time for cooling to a required temperature.

In a specific implementation process, a bypass pipeline is also arranged between the gas outlet of the gas-liquid separator 201 of the first stage refrigeration cycle and the inlet of the compressor 204. The bypass pipeline is provided with an expansible vessel 206 and a bypass control member 207. The bypass control member 207 is arranged at one side of the inlet and/or one side of the outlet of the expansible vessel 206.

In the embodiment shown in Fig. 5, the bypass control member 207 is arranged at one side of the inlet of the expansible vessel 206, and the bypass control member 207 is opened or closed to achieve the conditions for an evaporation pressure.

In a specific implementation process, the bypass control member 207 is a solenoid valve.

In one embodiment, the bypass pipeline is further provided with a capillary tube 208 which is arranged at one side of the outlet of the expansible vessel 206. A filter drier 209 is arranged between the condenser 205 and the inlet of the gas-liquid separator 201 of the first stage refrigeration cycle.

In one embodiment, one side of the liquid outlet of each gas-liquid separator is provided with a throttle valve.

As shown in Fig. 4, in the pre-cooling device referred to in this embodiment, a main control member 9 and a filter drier 10 is arranged between the gas inlet port 8 and the pressure regulating assembly 1. The main control member 9 is a solenoid valve, and specifically a normally closed solenoid valve.

This example further provides a cryotherapy system, comprising a cryoprobe, and a pre-cooling device for cryotherapy, wherein the pre-cooling device communicates with the cryoprobe and relates to the above optional solutions.

In conclusion, the pre-cooling device for cryotherapy and the cryotherapy system provided by this example realize precooling to multiple temperatures of different levels by virtue of a single-stage-compression auto-cascade refrigeration system assembly with at least three-stage refrigeration cycles, and can meet a variety of requirements of different operations and different gases.

Also, for precooling to temperatures of different levels, the pressure regulating assembly of the present invention may be configured to control the pressure of the gas outlet end of the pressure regulating assembly according to the current pre-cooled branch vent, thereby automatically and timely controlling the ventilation pressure of the pre-cooling device and maintaining the stability of the pressure as well during pre-cooling of different levels. In addition, by automatic regulation of the ventilation pressure, it is beneficial to increase the refrigeration speed through a matched ventilation pressure, thereby helping to avoid the problem about a too long pre-cooling time.

In an optional solution of this example, the ventilation pressure may be regulated according to different current temperatures and regulation demands in combination with changes of the temperature information.

Further, when the temperature represented by the temperature information does not reach a target temperature, the pressure of the gas outlet end of the pressure regulating assembly is controlled to be matched with the first target pressure information; when the temperature represented by the temperature information reaches the target temperature, the pressure is controlled to drop to a value matched with the second target pressure information; and therefore a gas is effectively saved under a condition that the gas reaches a required temperature.

In an optional solution of this example, double times of pre-cooling is also realized through a return gas pre-cooling assembly and a single-stage-compression auto-cascade refrigeration system assembly, thereby helping to further reduce the refrigeration temperature and effectively increase the refrigeration speed.

In an optional solution of this example, multi-stage refrigeration cycles are formed through gas-liquid separators and condensation evaporators, multi-stage refrigeration in a wide temperature range is realized, the refrigeration speed may be effectively increased as well, and the problem about a too long pre-cooling time is avoided.

In the end, it should be noted that the above examples are merely intended to describe technical solutions of the present invention rather than to limit them; although the present invention has been described in detail with reference to the foregoing examples, those of ordinary skill in the art should understand that: it is still possible to modify the technical solutions described in the foregoing examples, or equivalently substitute some or all of technical features; and these modifications or substitutions do not make the nature of the corresponding technical solutions depart from the scope of the technical solutions of the examples of the present invention.

## Claims

1. A pre-cooling device for cryotherapy, comprising a gas inlet port(8), at least three vent branches, a single-stage-compression auto-cascade refrigeration system assembly(2) and a pressure regulating assembly, wherein the gas inlet port(8) communicates with a gas inlet end of the pressure regulating assembly(1); a gas outlet end of the pressure regulating assembly(1) is connected to gas inlet ends of the at least three vent branches respectively; a gas inlet end of a cryoprobe connector(4) for a cryoprobe is connected to gas outlet ends of the at least three vent branches respectively;
the single-stage-compression auto-cascade refrigeration system assembly(2) comprises at least three-stage refrigeration cycles (200); for at least some of the refrigeration cycles (200), a refrigeration temperature thereof is lower than that of the previous stage refrigeration cycle;
each refrigeration cycle(200) is capable of exchanging heat with a gas flowing in the corresponding vent branch to pre-cool the gas flowing in this vent branch; each vent branch is provided with a branch control member(3) configured to control connection and disconnection of this vent branch; and
the pressure regulating assembly(1) is configured to control a pressure of the gas outlet end of the pressure regulating assembly(1), the pressure of the gas outlet end of the pressure regulating assembly(1) is associated with a vent branch to be pre-cooled, and for at least two vent branches, the gas outlet end of the pressure regulating assembly(1) has different pressures during pre-cooling;
wherein the pressure regulating assembly(1) is configured to control the pressure of the gas outlet end of the pressure regulating assembly(1) according to first pressure information and/or second pressure information, wherein the first pressure information represents a pressure of a gas inlet end of the cryoprobe connector(4), and the second pressure information represents a pressure of the gas inlet end of the pressure regulating assembly(1);
the pressure regulating assembly(1) comprises a proportional decompression valve(102) and a controller(101); the gas inlet end of the cryoprobe connector(4) is connected with a first pressure sensor(103) configured to detect the first pressure information; a gas inlet end of the proportional decompression valve(102) is connected with a second pressure sensor(104) configured to detect the second pressure information; a gas outlet end of the proportional decompression valve(102) is connected with a third pressure sensor(105); and
the controller(101) is connected with the first pressure sensor(103), the second pressure sensor(104) and the third pressure sensor(105) respectively, and is configured to control the proportional decompression valve(102) according to information detected by the first pressure sensor(103), the second pressure sensor(104) and the third pressure sensor(105).

2. The pre-cooling device according to claim 1, wherein the pressure regulating assembly(1) is configured to, after the vent branch to be pre-cooled is determined, determine target pressure information of the gas outlet end of the pressure regulating assembly(1) according to a preset corresponding relation between the vent branch and pressure information, and control the pressure of the gas outlet end of the pressure regulating assembly(1) according to the target pressure information.

3. The pre-cooling device according to any one of claims 1 to 2, wherein the pressure regulating assembly(1) is configured to control the pressure of the gas outlet end of the pressure regulating assembly(1) according to temperature information, wherein the temperature information comprises first temperature information and/or second temperature information; the first temperature information represents temperatures of the vent branches; and the second temperature information represents a temperature of the gas inlet end of the cryoprobe connector(4).

4. The pre-cooling device according to claim 3, wherein the first temperature information represents a temperature of a gas refrigerated by the corresponding refrigeration cycle(200) in the corresponding vent branch; and
after the temperature represented by the second temperature information is equal to a required temperature and the pressure of the gas outlet end of the pressure regulating assembly(1) reaches a target pressure, the pressure regulating assembly(1) is configured to control the pressure of the gas outlet end of the pressure regulating assembly(1) to be changed according to a change of the first temperature information, and maintain the temperature represented by the second temperature information to be equal to the required temperature.

5. The pre-cooling device according to claim 3, wherein in a control mode, if the temperature represented by the temperature information does not reach a preset target temperature, the pressure regulating assembly(1) is configured to control the pressure of the gas outlet end of the pressure regulating assembly(1) to be matched with the first target pressure information corresponding to the vent branch to be pre-cooled; and after the temperature represented by the temperature information reaches the target temperature and is kept for a preset target time, the pressure of the gas outlet end of the pressure regulating assembly(1) is controlled to drop to a value matched with the second target pressure information of the vent branch to be pre-cooled.

6. The pre-cooling device according to any one of claims 1 to 2, further comprising a return gas pre-cooling assembly, wherein the return gas pre-cooling assembly comprises a gas return channel and a return gas pre-cooling control member(6) which is arranged in the gas return channel; a gas inlet end of the gas return channel communicates with a gas return end of the cryoprobe connector(4); such that a gas circulating in the gas return channel is capable of exchanging heat with a gas circulating in the at least three vent branches to pre-cool the gas circulating in the at least three vent branches; and
the gas circulating in the vent branches first exchanges heat with the gas circulating in the gas return channel to achieve first pre-cooling and then exchanges heat with the corresponding refrigeration cycle(200) to achieve second pre-cooling.

7. The pre-cooling device according to any one of claims 1 to 2, wherein in at least some of the refrigeration cycles(200), a working medium for pre-cooling in each refrigeration cycle(200) is obtained by cooling a working medium for pre-cooling in the previous stage refrigeration cycle(200); each of the at least some of the refrigeration cycles(200) is provided with a condensation evaporator(202) and a gas-liquid separator(201);
excluding the first stage of the at least three-stage refrigeration cycles(200), the gas-liquid separators(201) of the other refrigeration cycles(200) are all used for gas and liquid separation of a gaseous working medium discharged from the previous stage refrigeration cycle(200) to implement cooling; and the condensation evaporator(202) in each refrigeration cycle(200) is configured to control a gaseous working medium discharged from the gas-liquid separator(201) of the same stage refrigeration cycle(200), a gas circulating in the corresponding vent branch and a liquid working medium discharged from the gas-liquid separator(201) of the same stage refrigeration cycle(200) to exchange heat with one another.

8. The pre-cooling device according to claim 7, wherein the first stage refrigeration cycle(200) is provided with a compressor(204), a condenser(205), a condensation evaporator(202) and a gas-liquid separator(201), and the last stage of the at least three-stage refrigeration cycles(200) is provided with an evaporator(203); and
a first input end and a first output end of the evaporator(203) are connected to the corresponding vent branch; a second input end of the evaporator(203) is connected with a gas outlet of the gas-liquid separator(201) of the previous stage refrigeration cycle(200); a second output end of the evaporator(203) is connected with a first inlet of the condensation evaporator(202) of the previous stage refrigeration cycle(200);
excluding the first stage refrigeration cycle(200) and the last stage refrigeration cycle(200), the inlet of the gas-liquid separator(201) of each stage of the other refrigeration cycle(200) communicates with a second outlet of the condensation evaporator(202) of the previous stage refrigeration cycle(200); a gas outlet of the gas-liquid separator(201) of each stage of the other refrigeration cycles(200) communicates with a second inlet of the condensation evaporator(202) of the same stage refrigeration cycle(200); a liquid outlet of the gas-liquid separator(201) of each stage of the other refrigeration cycles(200) communicates with a first inlet of the condensation evaporator(202) of the same stage refrigeration cycle(200); a first outlet of the condensation evaporator(202) of each stage of the other refrigeration cycle(200) communicates with a first inlet of the condensation evaporator(202) of the previous stage refrigeration cycle(200); a third inlet and a third outlet of the condensation evaporator(202) of each stage of the other refrigeration cycle(200) are respectively connected into the corresponding vent branch; and
the outlet of the compressor(204) communicates with the inlet of the condenser(205); the outlet of the condenser(205) communicates with the inlet of the gas-liquid separator(201) of the first stage refrigeration cycle(200); the liquid outlet of the gas-liquid separator(201) of the first stage refrigeration cycle(200) communicates with the first inlet of the condensation evaporator(202) of the first stage refrigeration cycle(200); the gas outlet of the gas-liquid separator(201) of the first stage refrigeration cycle(200) communicates with the second inlet of the condensation evaporator(202) of the first stage refrigeration cycle(200); the first outlet of the condensation evaporator(202) of the first stage refrigeration cycle(200) communicates with the inlet of the compressor(204); and the third inlet and the third outlet of the condensation evaporator(202) of the first stage refrigeration cycle(200) are respectively connected into the corresponding vent branch.

9. The pre-cooling device according to claim 8, wherein a bypass pipeline is arranged between the gas outlet of the gas-liquid separator(201) of the first stage refrigeration cycle(200) and the inlet of the compressor(204); the bypass pipeline is provided with an expansible vessel(206) and a bypass control member(207); and the bypass control member(207) is arranged at one side of the inlet and/or one side of the outlet of the expansible vessel(206).

10. A cryotherapy system, comprising a cryoprobe, and the pre-cooling device for cryotherapy according to any one of claims 1 to 9, wherein the pre-cooling device communicates with the cryoprobe.

## Patentansprüche

1. Vorkühlvorrichtung für die Kryotherapie, wobei die Vorkühlvorrichtung einen Gaseinlassanschluss (8), wenigstens drei Entlüftungszweige, eine Autokaskaden-Kühlsystemanordnung (2) mit einstufiger Verdichtung und eine Druckregulierungsanordnung umfasst, wobei der Gaseinlassanschluss (8) mit einem Gaseinlass-Ende der Druckregulierungsanordnung (1) in Verbindung steht; wobei ein Gasauslass-Ende der Druckregulierungsanordnung (1) jeweils mit Gaseinlass-Enden der wenigstens drei Entlüftungszweige verbunden ist; wobei ein Gaseinlass-Ende eines Kryosondenverbinders (4) für eine Kryosonde jeweils mit Gasauslass-Enden der wenigstens drei Entlüftungszweige verbunden ist;
wobei die Autokaskaden-Kühlsystemanordnung (2) mit einstufiger Verdichtung wenigstens dreistufige Kältekreisläufe (200) umfasst; wobei für wenigstens einige der Kältekreisläufe (200) eine Kühltemperatur davon niedriger als die des Kältekreislaufs der vorhergehenden Stufe ist;
wobei jeder Kältekreislauf (200) in der Lage ist, mit einem in dem entsprechenden Entlüftungszweig strömenden Gas Wärme auszutauschen, um das in diesem Entlüftungszweig strömende Gas vorzukühlen; wobei jeder Entlüftungszweig mit einem Zweigregulierungselement (3) versehen ist, das dafür konfiguriert ist, die Verbindung und Trennung dieses Entlüftungszweigs zu regulieren; und
wobei die Druckregulierungsanordnung (1) dafür konfiguriert ist, einen Druck des Gasauslass-Endes der Druckregulierungsanordnung (1) zu regulieren, wobei der Druck des Gasauslass-Endes der Druckregulierungsanordnung (1) einem vorzukühlenden Entlüftungszweig zugeordnet ist und wobei das Gasauslass-Ende der Druckregulierungsanordnung (1) für wenigstens zwei Entlüftungszweige während des Vorkühlens unterschiedliche Drücke aufweist;
wobei die Druckregulierungsanordnung (1) dafür konfiguriert ist, den Druck des Gasauslass-Endes der Druckregulierungsanordnung (1) in Übereinstimmung mit ersten Druckinformationen und/oder zweiten Druckinformationen zu regulieren, wobei die ersten Druckinformationen einen Druck eines Gaseinlass-Endes des Kryosondenverbinders (4) repräsentieren und die zweiten Druckinformationen einen Druck des Gaseinlass-Endes der Druckregulierungsanordnung (1) repräsentieren;
wobei die Druckregulierungsanordnung (1) ein Proportionalentspannungsventil (102) und eine Regulierungseinheit (101) umfasst; wobei das Gaseinlass-Ende des Kryosondenverbinders (4) mit einem ersten Drucksensor (103) verbunden ist, der dafür konfiguriert ist, die ersten Druckinformationen zu detektieren; wobei ein Gaseinlass-Ende des Proportionalentspannungsventils (102) mit einem zweiten Drucksensor (104) verbunden ist, der dafür konfiguriert ist, die zweiten Druckinformationen zu detektieren; wobei ein Gasauslass-Ende des Proportionalentspannungsventils (102) mit einem dritten Drucksensor (105) verbunden ist; und
wobei die Regulierungseinheit (101) jeweils mit dem ersten Drucksensor (103), mit dem zweiten Drucksensor (104) und mit dem dritten Drucksensor (105) verbunden ist und dafür konfiguriert ist, das Proportionalentspannungsventil (102) in Übereinstimmung mit Informationen zu regulieren, die durch den ersten Drucksensor (103), durch den zweiten Drucksensor (104) und durch den dritten Drucksensor (105) detektiert werden.

2. Vorkühlvorrichtung nach Anspruch 1, wobei die Druckregulierungsanordnung (1) dafür konfiguriert ist, nachdem der vorzukühlende Entlüftungszweig bestimmt worden ist, Solldruckinformationen des Gasauslass-Endes der Druckregulierungsanordnung (1) in Übereinstimmung mit einer im Voraus festgelegten entsprechenden Beziehung zwischen dem Entlüftungszweig und Druckinformationen zu bestimmen und den Druck des Gasauslass-Endes der Druckregulierungsanordnung (1) in Übereinstimmung mit den Solldruckinformationen zu regulieren.

3. Vorkühlvorrichtung nach einem der Ansprüche 1 bis 2, wobei die Druckregulierungsanordnung (1) dafür konfiguriert ist, den Druck des Gasauslass-Endes der Druckregulierungsanordnung (1) in Übereinstimmung mit Temperaturinformationen zu regulieren, wobei die Temperaturinformationen erste Temperaturinformationen und/oder zweite Temperaturinformationen umfassen; wobei die ersten Temperaturinformationen Temperaturen der Entlüftungszweige repräsentieren; und wobei die zweiten Temperaturinformationen eine Temperatur des Gaseinlass-Endes des Kryosondenverbinders (4) repräsentieren.

4. Vorkühlvorrichtung nach Anspruch 3, wobei die ersten Temperaturinformationen eine Temperatur eines durch den entsprechenden Kältekreislauf (200) in dem entsprechenden Entlüftungszweig gekühlten Gases repräsentieren; und
die Druckregulierungsanordnung (1) dafür konfiguriert ist, den Druck des Gasauslass-Endes der Druckregulierungsanordnung (1) so zu regulieren, dass er in Übereinstimmung mit einer Änderung der ersten Temperaturinformationen geändert wird und dass die durch die zweiten Temperaturinformationen repräsentierte Temperatur gleich der geforderten Temperatur gehalten wird, nachdem die durch die zweiten Temperaturinformationen repräsentierte Temperatur gleich einer geforderten Temperatur ist und der Druck des Gasauslass-Endes der Druckregulierungsanordnung (1) einen Solldruck erreicht hat.

5. Vorkühlvorrichtung nach Anspruch 3, wobei in einer Regulierungsbetriebsart, falls die durch die Temperaturinformationen repräsentierte Temperatur eine im Voraus festgelegte Solltemperatur nicht erreicht, die Druckregulierungsanordnung (1) dafür konfiguriert ist, den Druck des Gasauslass-Endes der Druckregulierungsanordnung (1) dafür zu regulieren, dass er an die ersten Solldruckinformationen, die dem vorzukühlenden Entlüftungszweig entsprechen, angepasst ist; und nachdem die durch die Temperaturinformationen repräsentierte Temperatur die Solltemperatur erreicht hat und für eine im Voraus festgelegte Sollzeit gehalten worden ist, der Druck des Gasauslass-Endes der Druckregulierungsanordnung (1) dafür reguliert wird, auf einen Wert zu fallen, der an die zweiten Solldruckinformationen des vorzukühlenden Entlüftungszweigs angepasst ist.

6. Vorkühlvorrichtung nach einem der Ansprüche 1 bis 2, die ferner eine Rückgas-Vorkühlanordnung umfasst, wobei die Rückgas-Vorkühlanordnung einen Gasrückkanal und ein Rückgas-Vorkühl-Regulierungselement (6), das in dem Gasrückkanal angeordnet ist, umfasst; wobei ein Gaseinlass-Ende des Gasrückkanals mit einem Gasrückstromende des Kryosondenverbinders (4) in Verbindung steht; sodass ein Gas, das in dem Gasrückkanal umgewälzt wird, in der Lage ist, mit einem Gas, das in den wenigstens drei Entlüftungszweigen umgewälzt wird, Wärme auszutauschen, um das Gas, das in den wenigstens drei Entlüftungszweigen umgewälzt wird, vorzukühlen; und
das Gas, das in den Entlüftungszweigen umgewälzt wird, zunächst Wärme mit dem Gas austauscht, das in dem Gasrückkanal umgewälzt wird, um eine ersten Vorkühlung zu erzielen, und daraufhin Wärme mit dem entsprechenden Kältekreislauf (200) austauscht, um eine zweite Vorkühlung zu erzielen.

7. Vorkühlvorrichtung nach einem der Ansprüche 1 bis 2, wobei in wenigstens einigen der Kältekreisläufe (200) ein Arbeitsmedium zum Vorkühlen in jedem Kältekreislauf (200) durch Kühlen eines Arbeitsmediums zum Vorkühlen in dem Kältekreislauf (200) der vorhergehenden Stufe erhalten wird; wobei jeder der wenigstens einigen Kältekreisläufe (200) mit einem Kondensationsverdampfer (202) und mit einem Gas-Flüssigkeits-Abscheider (201) versehen ist;
wobei die Gas-Flüssigkeits-Abscheider (201) der anderen Kältekreisläufe (200) mit Ausnahme der ersten Stufe der wenigstens dreistufigen Kältekreisläufe (200) alle für die Gas- und Flüssigkeitstrennung eines gasförmigen Arbeitsmediums verwendet werden, das von dem Kältekreislauf (200) der vorhergehenden Stufe abgelassen wird, um eine Kühlung zu implementieren; und wobei der Kondensationsverdampfer (202) in jedem Kältekreislauf (200) dafür konfiguriert ist, ein von dem Gas-Flüssigkeits-Abscheider (201) des Kältekreislaufs (200) derselben Stufe abgelassenes gasförmiges Arbeitsmedium, ein in dem entsprechenden Entlüftungszweig umgewälztes Gas und ein von dem Gas-Flüssigkeits-Abscheider (201) des Kältekreislaufs (200) derselben Stufe abgelassenes flüssiges Arbeitsmedium zu regulieren, um Wärme miteinander auszutauschen.

8. Vorkühlvorrichtung nach Anspruch 7, wobei der Kältekreislauf (200) der ersten Stufe mit einem Kompressor (204), mit einem Kondensator (205), mit einem Kondensationsverdampfer (202) und mit einem Gas-Flüssigkeits-Abscheider (201) versehen ist und wobei die letzte Stufe der wenigstens dreistufigen Kältekreisläufe (200) mit einem Verdampfer (203) versehen ist; und
wobei ein erstes Eingangsende und ein erstes Ausgangsende des Verdampfers (203) mit dem entsprechenden Entlüftungszweig verbunden sind; wobei ein zweites Eingangsende des Verdampfers (203) mit einem Gasauslass des Gas-Flüssigkeits-Abscheiders (201) des Kältekreislaufs (200) der vorhergehenden Stufe verbunden ist; wobei ein zweites Ausgangsende des Verdampfers (203) mit einem ersten Einlass des Kondensationsverdampfers (202) des Kältekreislaufs (200) der vorhergehenden Stufe verbunden ist;
der Einlass des Gas-Flüssigkeits-Abscheiders (201) jeder Stufe des anderen Kältekreislaufs (200) mit Ausnahme des Kältekreislaufs (200) der ersten Stufe und des Kältekreislaufs (200) der letzten Stufe mit einem zweiten Auslass des Kondensationsverdampfers (202) des Kältekreislaufs (200) der vorhergehenden Stufe in Verbindung steht; ein Gasauslass des Gas-Flüssigkeits-Abscheiders (201) jeder Stufe der anderen Kältekreisläufe (200) mit einem zweiten Einlass des Kondensationsverdampfers (202) des Kältekreislaufs (200) derselben Stufe in Verbindung steht; ein Flüssigkeitsauslass des Gas-Flüssigkeits-Abscheiders (201) jeder Stufe der anderen Kältekreisläufe (200) mit einem ersten Einlass des Kondensationsverdampfers (202) des Kältekreislaufs (200) derselben Stufe in Verbindung steht; ein erster Auslass des Kondensationsverdampfers (202) jeder Stufe des anderen Kältekreislaufs (200) mit einem ersten Einlass des Kondensationsverdampfers (202) des Kältekreislaufs (200) der vorhergehenden Stufe in Verbindung steht; ein dritter Einlass und ein dritter Auslass des Kondensationsverdampfers (202) jeder Stufe des anderen Kältekreislaufs (200) jeweils mit einem entsprechenden Entlüftungszweig verbunden sind; und
der Auslass des Kompressors (204) mit dem Einlass des Kondensators (205) in Verbindung steht; der Auslass des Kondensators (205) mit dem Einlass des Gas-Flüssigkeits-Abscheiders (201) des Kältekreislaufs (200) der ersten Stufe in Verbindung steht; der Flüssigkeitsauslass des Gas-Flüssigkeits-Abscheiders (201) des Kältekreislaufs (200) der ersten Stufe mit dem ersten Einlass des Kondensationsverdampfers (202) des Kältekreislaufs (200) der ersten Stufe in Verbindung steht; der Gasauslass des Gas-Flüssigkeits-Abscheiders (201) des Kältekreislaufs (200) der ersten Stufe mit dem zweiten Einlass des Kondensationsverdampfers (202) des Kältekreislaufs (200) der ersten Stufe in Verbindung steht; der erste Auslass des Kondensationsverdampfers (202) des Kältekreislaufs (200) der ersten Stufe mit dem Einlass des Kompressors (204) in Verbindung steht; und der dritte Einlass und der dritte Auslass des Kondensationsverdampfers (202) des Kältekreislaufs (200) der ersten Stufe jeweils mit dem entsprechenden Entlüftungszweig verbunden sind.

9. Vorkühlvorrichtung nach Anspruch 8, wobei zwischen dem Gasauslass des Gas-Flüssigkeits-Abscheiders (201) des Kältekreislaufs (200) der ersten Stufe und dem Einlass des Kompressors (204) eine Umgehungsrohrleitung angeordnet ist; wobei die Umgehungsrohrleitung mit einem dehnbaren Gefäß (206) und mit einem Umgehungsregulierungselement (207) versehen ist; und wobei das Umgehungsregulierungselement (207) auf einer Seite des Einlasses und/oder auf einer Seite des Auslasses des dehnbaren Gefäßes (206) angeordnet ist.

10. Kryotherapiesystem, das eine Kryosonde und die Vorkühlvorrichtung für die Kryotherapie nach einem der Ansprüche 1 bis 9 umfasst, wobei die Vorkühlvorrichtung mit der Kryosonde in Verbindung steht.

## Revendications

1. Dispositif de pré-refroidissement pour la cryothérapie, comprenant un port d'entrée de gaz (8), au moins trois branches de ventilation, un système de réfrigération auto-cascade à compression à un étage (2) et un ensemble de régulation de la pression, dans lequel le port d'entrée de gaz (8) communique avec une extrémité d'entrée de gaz de l'ensemble de régulation de la pression (1), une extrémité de sortie de gaz de l'ensemble de régulation de la pression (1) est connectée respectivement aux extrémités d'entrée de gaz des au moins trois branches de ventilation; une extrémité de sortie de gaz de l'ensemble de régulation de pression (1) est respectivement connectée aux extrémités d'entrée de gaz d'au moins trois branches de ventilation; une extrémité d'entrée de gaz d'un connecteur de cryosonde (4) pour une cryosonde est connectée aux extrémités de sortie de gaz d'au moins trois branches de ventilation respectivement ;
l'ensemble de système de réfrigération auto-cascade à compression à un étage (2) comprend au moins des cycles de réfrigération à trois étages (200) ; pour au moins certains des cycles de réfrigération (200), leur température de réfrigération étant inférieure à celle du cycle de réfrigération de l'étage précédent ;
chaque cycle de réfrigération (200) est capable d'échanger de la chaleur avec un gaz circulant dans la branche de ventilation correspondante pour pré-refroidir le gaz circulant dans cette branche de ventilation ; chaque branche de ventilation est pourvue d'un élément de commande de branche (3) configuré pour commander la connexion et la déconnexion de cette branche de ventilation ; et
l'ensemble de régulation de pression (1) est configuré pour commander une pression de l'extrémité de sortie de gaz de l'ensemble de régulation de pression (1), la pression de l'extrémité de sortie de gaz de l'ensemble de régulation de pression (1) est contrôlée par un élément de commande de branche (3) configuré pour contrôler la connexion et la déconnexion de cette branche de ventilation. l'ensemble de régulation de pression (1) est associée à une branche de ventilation à pré-refroidir, et pour au moins deux branches de ventilation, l'extrémité de sortie de gaz de l'ensemble de régulation de pression (1) a des pressions différentes pendant le pré-refroidissement ;
dans lequel l'ensemble de régulation de pression (1) est configuré pour contrôler la pression de l'extrémité de sortie de gaz de l'ensemble de régulation de pression (1) en fonction des premières informations de pression et/ou des secondes informations de pression, dans lequel les premières informations de pression représentant une pression d'une extrémité d'entrée de gaz du connecteur de cryosonde (4), et les secondes informations de pression représentant une pression de l'extrémité d'entrée de gaz de l'ensemble de régulation de pression (1) ;
l'ensemble de régulation de pression (1) comprend une valve de décompression proportionnelle (102) et un contrôleur (101) ; l'extrémité d'entrée de gaz du connecteur de cryosonde (4) est connectée à un premier capteur de pression (103) configuré pour détecter la première information de pression ; une extrémité d'entrée de gaz de la valve de décompression proportionnelle (102) est connectée à un deuxième capteur de pression (104) configuré pour détecter la deuxième information de pression ; une extrémité de sortie de gaz de la valve de décompression proportionnelle (102) est connectée à un troisième capteur de pression (105) ;
et le contrôleur (101) est respectivement connecté au premier capteur de pression (103), au deuxième capteur de pression (104) et au troisième capteur de pression (105) ; et il est configuré pour commander la soupape de décompression proportionnelle (102) en fonction des informations détectées par le premier capteur de pression (103), le deuxième capteur de pression (104) et le troisième capteur de pression de pression (105).

2. Dispositif de pré-refroidissement selon la revendication 1, dans lequel l'ensemble de régulation de pression (1) est configuré pour, après la détermination de la branche de ventilation à pré-refroidir, déterminer l'information de pression cible de l'extrémité de sortie de gaz de l'ensemble de régulation de pression (1) en fonction d'une relation de correspondance prédéfinie entre la branche de ventilation et l'information de pression, et contrôler la pression de l'extrémité de sortie de gaz de l'ensemble de régulation de pression (1) en fonction de l'information de pression cible.

3. Dispositif de pré-refroidissement selon l'une quelconques des revendications 1 à 2, dans lequel l'ensemble de régulation de pression (1) est configuré pour contrôler la pression de l'extrémité de sortie de gaz de l'ensemble de régulation de pression (1) en fonction des informations de température, dans lequel les informations de température comprennent les premières informations de température et/ou les secondes informations de température ; la première information de température représente des températures des branches de ventilation; la deuxième information de température représente une température de l'extrémité d'entrée de gaz du connecteur de cryosonde (4).

4. Dispositif de pré-refroidissement selon la revendication 3, dans lequel la première information de température représente une température d'un gaz réfrigéré par le cycle de réfrigération correspondant (200) dans la branche de ventilation correspondante ; et
une fois que la température représentée par la deuxième information de température est égale à une température requise et que la pression de l'extrémité de sortie de gaz de l'ensemble de régulation de pression (1) atteint une pression cible, l'ensemble de régulation de pression (1) est configuré pour contrôler la pression de l'extrémité de sortie de gaz de l'ensemble de régulation de pression (1) pour être modifiée en fonction d'un changement de la première information de température, et maintenir la température représentée par la deuxième information de température pour qu'elle soit égale à la température requise.

5. Dispositif de pré-refroidissement selon la revendication 3, dans lequel, dans un mode de contrôle, si la température représentée par l'information de température n'atteint pas une température cible prédéfinie, l'ensemble de régulation de pression (1) est configuré pour contrôler la pression de l'extrémité de sortie de gaz de l'ensemble de régulation de pression (1) afin qu'elle corresponde à la première information de pression cible correspondant à la branche de ventilation à pré-refroidir ; et après que la température représentée par l'information de température a atteint la température cible et est maintenue pendant une durée cible prédéfinie, la pression de l'extrémité de sortie de gaz de l'ensemble de régulation de pression (1) est contrôlée pour chuter à une valeur correspondant à la deuxième information de pression cible de la branche de ventilation à pré-refroidir.

6. Dispositif de pré-refroidissement selon l'une des revendications 1 à 2, comprenant en outre un ensemble de pré-refroidissement du gaz de retour, dans lequel l'ensemble de pré-refroidissement du gaz de retour comprend un canal de retour du gaz et un élément de commande de pré-refroidissement du gaz de retour (6) qui est disposé dans le canal de retour du gaz ; une extrémité d'entrée du gaz du canal de retour du gaz communique avec une extrémité de retour du gaz du connecteur de la cryosonde (4) ; de sorte qu'un gaz circulant dans le canal de retour de gaz est capable d'échanger de la chaleur avec un gaz circulant dans les au moins trois branches de ventilation pour pré-refroidir le gaz circulant dans les au moins trois branches de ventilation ; et le gaz circulant dans les branches de ventilation échange d'abord de la chaleur avec le gaz circulant dans le canal de retour de gaz pour réaliser un premier pré-refroidissement et échange ensuite de la chaleur avec le cycle de réfrigération (200) correspondant pour réaliser un second pré-refroidissement.

7. Dispositif de pré-refroidissement selon l'une des revendications 1 à 2, dans lequel dans au moins certains des cycles de réfrigération (200), un fluide de travail pour le pré-refroidissement dans chaque cycle de réfrigération (200) est obtenu en refroidissant un fluide de travail pour le pré-refroidissement dans le cycle de réfrigération(200) de l'étape précédente ; chacun des au moins certains des cycles de réfrigération (200) est pourvu d'un évaporateur de condensation (202) et d'un séparateur de gaz-liquide (201) ;
à l'exception du premier étage des cycles de réfrigération (200) à au moins trois étages, les séparateurs gaz-liquide (201) des autres cycles de réfrigération (200) sont tous utilisés pour la séparation gaz-liquide d'un fluide de travail gazeux évacué de l'étage précédent du cycle de réfrigération (200) afin de mettre en oeuvre le refroidissement ; et l'évaporateur de condensation (202) de chaque cycle de réfrigération (200) est configuré pour contrôler un fluide de travail gazeux évacué par le séparateur gaz-liquide (201) du même cycle de réfrigération (200), un gaz circulant dans la branche de ventilation correspondante et un fluide de travail liquide évacué par le séparateur gaz-liquide (201) du même cycle de réfrigération (200) afin d'échanger de la chaleur avec l'autre.

8. Dispositif de pré-refroidissement selon la revendication 7, dans lequel le cycle de réfrigération du premier étage (200) est équipé d'un compresseur (204), d'un condenseur (205), d'un évaporateur de condensation (202) et d'un séparateur gaz-liquide (201), et le dernier étage des cycles de réfrigération au moins à trois étages (200) est équipé d'un évaporateur (203) ; et
une première extrémité d'entrée et une première extrémité de sortie de l'évaporateur (203) sont connectées à la branche de ventilation correspondante ; une deuxième extrémité d'entrée de l'évaporateur (203) est connectée à une sortie de gaz du séparateur gaz-liquide (201) du cycle de réfrigération (200) de l'étage précédent ; une deuxième extrémité de sortie de l'évaporateur (203) est connectée à une première entrée de l'évaporateur de condensation (202) du cycle de réfrigération (200) de l'étage précédent ;
à l'exception du cycle de réfrigération du premier étage (200) et du cycle de réfrigération du dernier étage (200), l'entrée de l'évaporateur de condensation (200), l'entrée du séparateur gaz-liquide (201) de chaque étage de l'autre cycle de réfrigération (200) communique avec une deuxième sortie de l'évaporateur de condensation (202) du cycle de réfrigération (200) de l'étage précédent ; une sortie de gaz du séparateur gaz-liquide (201) de chaque étage des autres cycles de réfrigération (200) communique avec une deuxième entrée de l'évaporateur de condensation (202) du même cycle de réfrigération (200) ; une sortie de liquide du séparateur gaz-liquide (201) de chaque étage des autres cycles de réfrigération (200) communique avec une première entrée de l'évaporateur de condensation (202) du même cycle de réfrigération (200) ; une première sortie de l'évaporateur de condensation (202) de chaque étage de l'autre cycle de réfrigération (200) communique avec une première entrée de l'évaporateur de condensation (202) de l'étage précédent du cycle de réfrigération (200) ; une troisième entrée et une troisième sortie de l'évaporateur de condensation (202) de chaque étage de l'autre cycle de réfrigération (200) sont respectivement connectées à la branche d'évent correspondante ; et
la sortie du compresseur (204) communique avec l'entrée du condenseur (205) ; la sortie du condenseur (205) communique avec l'entrée du séparateur gaz-liquide (201) du cycle de réfrigération du premier étage (200) ; la sortie de liquide du séparateur gaz-liquide La sortie liquide du séparateur gaz-liquide (201) du premier cycle de réfrigération (200) communique avec la première entrée de l'évaporateur de condensation (202) du premier cycle de réfrigération (200) ; la sortie gaz du séparateur gaz-liquide (201) du premier cycle de réfrigération (200) communique avec la deuxième entrée de l'évaporateur de condensation (202) du premier cycle de réfrigération (200) ; la première sortie de l'évaporateur de condensation (202) du premier étage du cycle de réfrigération (200) communique avec l'entrée du compresseur (204) ; et la troisième entrée et la troisième sortie de l'évaporateur de condensation (202) du premier étage du cycle de réfrigération (200) sont respectivement connectées à la branche d'aération correspondante.

9. Dispositif de pré-refroidissement selon la revendication 8, dans lequel une conduite de dérivation est disposée entre la sortie de gaz du séparateur gaz-liquide (201) du cycle de réfrigération de premier étage (200) et l'entrée du compresseur (204) ; la conduite de dérivation est pourvue d'un récipient expansible (206) et d'un élément de commande de dérivation (207) ; et l'élément de commande de dérivation (207) est disposé d'un côté de l'entrée et/ou d'un côté de la sortie du récipient expansible (206).

10. Système de cryothérapie comprenant une cryosonde et le dispositif de pré-refroidissement pour la cryothérapie selon l'une des revendications 1 à 9, dans lequel le dispositif de pré-refroidissement communique avec la cryosonde.
